# EUROPEAN PATENT SPECIFICATION

(11) **EP 2 554 193 B1**
(45) Date of publication and mention of the grant of the patent: **30.08.2023**
(21) Application number: 11765341.0
(22) Date of filing: 16.03.2011
(51) Int. Cl.: A61M 5/00, A61B 5/00, A61M 5/142

(54) **INTEGRATED CIRCUIT AND MEDICAL INSTRUMENT USING SAME**
INTEGRIERTER SCHALTKREIS UND MEDIZINISCHES INSTRUMENT DAMIT
CIRCUIT INTÉGRÉ ET INSTRUMENT MÉDICAL L'UTILISANT

(30) Priority: 31.03.2010 JP 2010080224
(43) Date of publication of application: 06.02.2013
(73) Proprietor: Terumo Kabushiki Kaisha, Tokyo 151-0072 (JP)
(72) Inventor: MORITA, Takashi, Ashigarakami-gun Kanagawa 259-0151 (JP); SUGAWARA, Yoshihisa, Ashigarakami-gun Kanagawa 259-0151 (JP); IWATA, Takeharu, Ashigarakami-gun Kanagawa 259-0151 (JP)
(74) Representative: TBK
(86) International application number: PCT/JP2011/056218
(87) International publication number: WO 2011/125439

(56) References cited:
- WO-A1-03/105931
- WO-A2-2007/121170
- WO-A2-2009/030934
- JP-A- H1 080 401
- JP-A- 2006 503 597
- JP-A- 2009 148 375
- JP-B2- 4 298 266
- HUI CAO ET AL: "A Novel Non-invasive Medical Measurement Based on Parallel Neural Computation Supported by the Grid", NATURAL COMPUTATION, 2008. ICNC '08. FOURTH INTERNATIONAL CONFERENCE ON, IEEE, PISCATAWAY, NJ, USA, 18 October 2008 (2008-10-18), pages 260-266, XP031358299, ISBN: 978-0-7695-3304-9

## Description

### TECHNICAL FIELD

The present invention relates to an integrated circuit and a medical device using the same.

### BACKGROUND ART

Heretofore, an internal circuit for medical devices is constituted by a hybrid packaging in which electronic components such as a microcomputer and an operational amplifier are mounted on a wiring substrate.

A document listed below (patent document 1) describes a technique for achieving a cost reduction of components by integrating a plurality of components into a single integrated circuit (hereinafter, referred to as "made into one chip").

### RELATED ART LITERATURE

### PATENT DOCUMENT

Patent document 1: JP-A-2001-212098

Document JP H10 80401 A discloses a small-sized sphygomanometer which makes both of blood pressure measurement and electrocardiogram measurement possible and facilitates the judgment of the state of a person to be measured at the time of blood pressure measurement.

Document WO 2009/030934 A2 discloses that by providing illumination over a range of excitation frequencies or wavelengths and using a multi time gating technique with regard to returned light, images are created which provide a spectral depth due to the varying scatter and absorption effects at the different excitation wavelengths. These images can be consolidated in order to provide a substantially real time view of pulsing action dependent upon blood oxygen saturation within the blood circulation system.

Document WO 2007/121170 A2 discloses improved devices for processing data from one or more physiological sensors based on parallel processing. The provided devices are small, low power, and readily configurable for use in most physiological monitoring applications. The provided devices are used for ambulatory monitoring of a subject's cardio-respiratory systems, and in particular, process data from one or more respiratory inductive plethysmographic sensors. Other relevant prior art include HUI CAO ET AL: "A Novel Non-invasive Medical Measurement Based on Parallel Neural Computation Supported by the Grid", NATURAL COMPUTATION, 2008. ICNC '08. IEEE FOURTH CONFERENCE ON INTERNATIONAL CONFERENCE, 18 October 2008, pages 260-266; and WO 03/105931 A1.

### DISCLOSURE OF THE INVENTION

### PROBLEMS TO BE SOLVED BY THE INVENTION

However, the integrated circuit made into one chip by above-mentioned conventional technique is customized as dedicated components for one device, therefore, it cannot be used as common components to be mounted on other devices in which the functions required for the integrated circuit to be used are different. Therefore, there is a problem that it is difficult to achieve a further cost reduction according to communalizing components in a plurality of devices.

The present invention is made in order to solve such a problem, and an object of the present invention is to achieve a further cost reduction of components for medical device and a medical device which uses the components.

### MEANS TO SOLVE THE PROBLEMS

In order to solve the above-described problems, according to an aspect of the present invention, there is provided an integrated circuit as defined in claim 1. Further aspects and features of the present invention are set out in the dependent claims.

### EFFECT OF THE INVENTION

According to the integrated circuit of the present invention, a group of common components which is used for each of a plurality of medical devices is communalized as a platform integrated circuit, while an integrated circuit (hereinafter, referred to as "functional complement integrated circuit") which complements a part of functions of the platform integrated circuit is used together with the platform integrated circuit for high performance devices. Accordingly, it is possible to communalize the components of a plurality of medical devices, to expand coverage of target medical devices, and to reduce the chip size of the common integrated circuit, and therefore, it is possible to achieve a further cost reduction of the components for medical devices and the medical device using the same.
FIG. 1 is a drawing illustrating a functional block diagram of a syringe pump and an infusion pump among medical devices used with the integrated circuit according to the embodiments of the present invention .
FIG. 2 is a drawing illustrating a functional block diagram of a blood glucose meter and a sphygmomanometer among medical devices used with the integrated circuit according to the embodiments of the present invention.
FIG. 3 is an explanatory diagram for explaining a functional block structure of a platform integrated circuit according to the embodiments of the present invention.
FIG. 4 is a drawing illustrating a manner of use of the platform integrated circuit 100 in the syringe pump.
FIG. 5 is a drawing illustrating a manner of use of the platform integrated circuit in the infusion pump.
FIG. 6 is a drawing illustrating a manner of use of the platform integrated circuit in the sphygmomanometer.
FIG. 7 is a drawing illustrating a manner of use of the platform integrated circuit in the blood glucose meter.

### PREFERRED MODES FOR CARRYING OUT THE INVENTION

Hereinafter, an integrated circuit and medical devices using the same according to the embodiments of the present invention will be described in detail with reference to drawings.

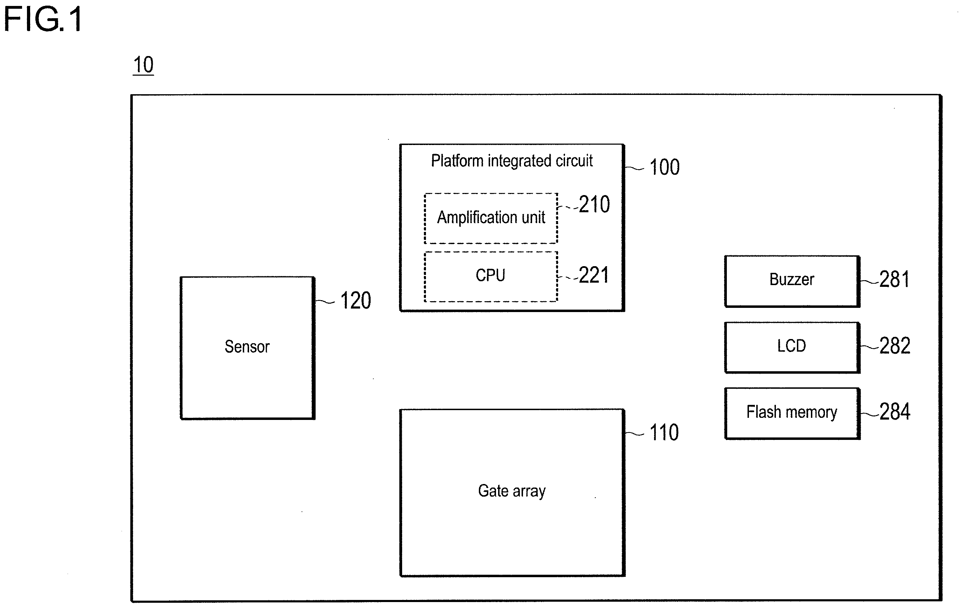

FIG. 1 is a drawing illustrating a functional block diagram of a syringe pump and an infusion pump among the medical devices used with the integrated circuit according to the embodiments of the present invention.

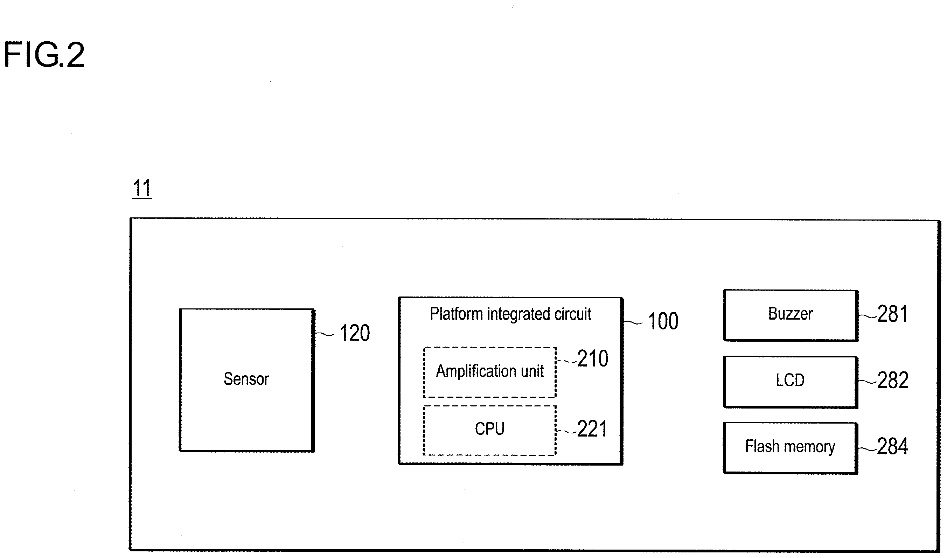

FIG. 2 is a drawing illustrating a functional block diagram of a blood glucose meter and a sphygmomanometer among the medical devices used with the integrated circuit according to the embodiments of the present invention.

Some functional blocks of the medical devices are omitted in FIG. 1 and FIG. 2.

As illustrated in FIG. 1 and FIG. 2, an integrated circuit 100 (hereinafter, referred to as "platform integrated circuit") according to the present embodiment is commonly used for the medical devices 10 and 11.

In the medical devices 10 and 11 illustrated in FIG. 1 and FIG. 2, when a platform integrated circuit 100 inputs a signal (hereinafter referred to as "detection signal") detected by the sensor 120 provided in each medical device, the detection signal is amplified by an amplification unit 210 included in the platform integrated circuit 100.

The medical devices 10 and 11 can be any of a syringe pump, an infusion pump, a blood glucose meter and a sphygmomanometer.

The sensor 120 is an occlusion pressure sensor in cases where the medical device 10 or 11 is the syringe pump and the infusion pump, is a pressure sensor and a microphone in cases where the medical device 10 or 11 is the sphygmomanometer, and is a blood glucose sensor in cases where the medical device 10 or 11 is the blood glucose meter.

A CPU (Central Processing Unit, arithmetic unit) 221 included in the platform integrated circuit 100 calculates based on the amplified detection signal, calculates a detection result, and controls the medical devices 10 and 11. The detection result is recorded on a flash memory 284 provided in the medical devices 10 and 11, and is displayed on an LCD (Liquid Crystal Display) 282. Moreover, when determining a completion of measurement and detecting abnormalities, a control can be made in which a buzzer 281 provided in the medical devices 10 and 11 is sounded.

As illustrated in FIG. 1, a gate array 110 is used with the platform integrated circuit 100 for the medical device 10 among the devices in the present embodiment. On the other hand, as illustrated in FIG. 2, the gate array 110 is not used for the medical device 11 among the devices in the present embodiment.

Thus, the platform integrated circuit 100 according to the present embodiment can use the gate array 110 together, in accordance with the functions of the medical devices 10 and 11 which are used. The gate array 110 is constituted so as to complement a part of functions of the platform integrated circuit 100.

The gate array 110 may be, for example, a gate array customized bya change of wiring (for example, aluminum master slice manner), and may be a gate array programmably customized by the writing or re-writing of a logic circuit (for example, FPGA (Field Programmable Gate Array)). The gate array 110 can use one commonly-marketed as multi-use parts.

Generally, high function (high performance) arithmetic unit, that is, a CPU, which has high arithmetic processing performance is required for the integrated circuit used for the syringe pump and the infusion pump. On the other hand, with respect to the integrated circuit used for the sphygmomanometer and the blood glucose meter, it does not require as much high-performance CPU as used for the syringe pump and the infusion pump. Therefore, if an attempt was made to realize all functions of above-described four medical devices 10 and 11 by the CPU 221 of the platform integrated circuit 100, there was no choice but to have the functions of CPU in accordance with the syringe pump and the infusion pump, and this caused over performance in the CPU 221 of the platform integrated circuit 100 when used for the sphygmomanometer and the blood glucose meter. That is, this causes an increase of the chip size of the platform integrated circuit 100, and it comes difficult to realize effective cost reduction for components.

In view of above, in the platform integrated circuit 100 according to the present embodiment, the CPU has a function of relatively low arithmetic processing performance which is sufficient to achieve the function of the sphygmomanometer and the blood glucose meter. In cases where it is used for the syringe pump and the infusion pump, the CPU 221 of the platform integrated circuit 100 and the gate array 110 are connected, and the CPU 221 of the platform integrated circuit 100 and the gate array 110 perform a parallel processing to perform arithmetic processes. That is, the medical device 11 is used for the sphygmomanometer and the blood glucose meter, the medical device 10 is used for the syringe pump and the fusion pump, and in the medical device 10, the platform integrated circuit 100 is constituted so that the gate array 110 for complementing the functions of the CPU of the platform integrated circuit 100 by the parallel processing with the CPU of the platform integrated circuit 100 can be connected. The functions which the gate array 110 provided in the syringe pump and the infusion pump complement may be, for example, an air bubble detection function for detecting the air bubbles in a tube, an intravenous drip detection function for detecting an intravenous drip state, and a motor control function etc. and other functions are provided by the CPU 221.

In this way, according to the platform integrated circuit 100 of the present embodiment, the scale of CPU 221 of the platform integrated circuit 100 commonly used to a plurality of medical devices 10 and 11 can be made small, and this allows a reduction of a chip size and cost for components. It is desirable to constitute the platform integrated circuit 100 by a full-custom ASIC(Application Specific Integrated Circuit) in order to improve an effect of reducing the chip size, but it may be constituted by a semi-custom standard cell.

According to the platform integrated circuit 100 of the present embodiment, even if the functions of some components of the platform integrated circuit 100 is constituted so that these components have relatively low functions, application coverage is expandable to measuring devices which require functions higher than the functions of the platform integrated circuit 100. Therefore, the cost for components can be further reduced by volume efficiency.

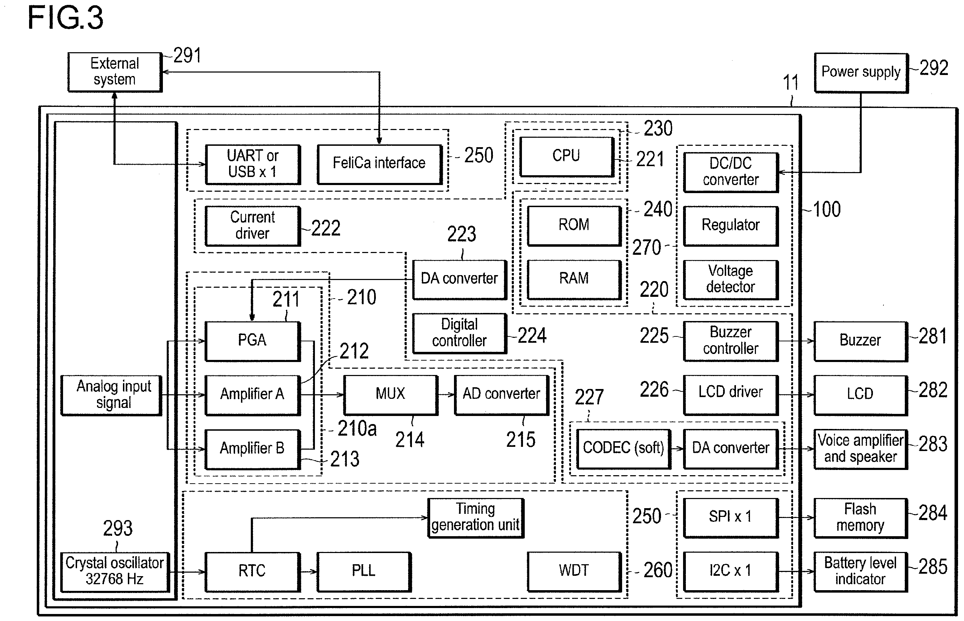

FIG. 3 is an explanatory diagram for explaining a functional block structure of the platform integrated circuit according to the embodiment of the present invention. In FIG. 3, the components of the medical devices 10 and 11 and external devices (a power supply 292 and an external system 291) connected to the medical devices 10 and 11 are also illustrated with the platform integrated circuit 100.

The medical device 10 can be used as the syringe pump and the infusion pump for example, the medical device 11 can be used as the blood glucose meter and the sphygmomanometer for example, and the platform integrated circuit 100 of the present embodiment is used for the medical devices 10 and 11 in common, respectively. In FIG. 3, the gate array is not illustrated which is connected to the platform integrated circuit when used as the medical device 10.

As illustrated in FIG. 3, the platform integrated circuit 100 is made into one chip so that it can be commonly used to the syringe pump, the infusion pump, the blood glucose meter and the sphygmomanometer, and it includes a group of components (functional blocks) which are commonly required for a plurality of these medical devices 10 and 11. Accordingly, it is possible to reduce the cost for components and the cost for implementation due to making the components into one chip and communalization of components.

The platform integrated circuit 100 of the present embodiment includes, for example, a signal processing unit 210, a control unit 220, an arithmetic unit 230, a memory unit 240, an input-output unit 250, an internal clock unit 260, and a power supply unit 270.

The signal processing unit includes an amplification unit 210a, a multiplexer 214, and an AD converter 215.

The amplification unit 210a includes a PGA (Programmable Gain Amp) 211, an amplifier A212 and an amplifier B213, and amplifies an analog input signal from each external sensor arranged outside of the platform integrated circuit 100. The PGA 211 may be constituted as a differential amplifier with a variable amplification rate using three operational amplifiers and a resistor. The amplifier A212 and the amplifier B213 may be constituted as an amplifier having a single input using an operational amplifier, respectively.

The multiplexer 214 multiplexes in time division the analog input signal amplified by the PGA 211, the amplifier A212 and the amplifier B213 at the predetermined timing based on the internal clock generated by the internal clock unit 260.

In FIG. 3, the multiplexer 214 multiplexes only analog signal from the amplification unit 210a, but the multiplexer 214 may be constituted so that a plurality of analog input signals including the analog input signal (not illustrated) which does not pass the amplification unit 210a can be multiplexed. A multiplexer which can multiplex the signals of eight channels may be used as the multiplexer 214.

The AD converter 215 applies a digital conversion so that the analog input signal multiplexed by the multiplexer is converted into a digital signal.

The analog input signal which is multiplexed and applied the digital conversion can be stored in the flash memory 284 implemented outside of the platform integrated circuit 100, through a SPI (Serial Peripheral Interface).

The control unit 220 includes a CPU 221, a current driver 222, a DA converter 223, a digital controller 224, a buzzer controller 225, an LCD driver 226, and a voice unit 227.

The CPU 221 controls each component of the platform integrated circuit 100 based on programs (internal programs) stored in the memory unit. Moreover, the CPU221 serves as an arithmetic unit 230 and performs various calculation based on the programs stored in the memory unit 240.

The current driver 222 includes a constant current source circuit, and applies current to various sensors such as an occlusion pressure sensor mounted on the medical device.

The DA converter 223 converts the digital control signal from the CPU 221 or the digital controller 224 into an analog control signal, and sets a gain of the PGA 211 according to each function of the medical devices 10 and 11 by the analog control signal.

The buzzer controller 225 controls the buzzer 281 mounted on the medical device.

The LCD driver 226 controls the LCD 282 mounted on the medical device, and controls the image displayed on the LCD 282.

The voice unit 227 includes a CODEC (Coder/Decorder) and a DA converter, and multiplexes and applies a DA conversion to the digital sound signal stored in the memory unit 240, makes a voice amplifier which is mounted the medical device amplify the signal to output a voice from the speaker 283.

The arithmetic unit 230 is constituted by the CPU 221 and performs various calculations in accordance with instructions from the control unit 220.

The memory unit 240 includes a ROM (Read Only Memory) and a RAM (Random Access Memory) . The ROM is a non-volatile memory storage and stores the programs for each of medical devices 10 and 11. The programs for each of medical devices 10 and 11 may be installed into the ROM from the external system 291. The RAM may serves as a main memory unit which the CPU 221 directly accesses.

The input-output unit 250 can be constituted so as to include an SPI, an I2C (Inter-Integrated Circuit), a UART (Universal Asynchronous Receiver) or a USB (Universal Serial Bus), and a FeliCa interface. For example, the SPI and the I2C can be applied to interfaces with the flash memory 284 and the battery level indicator 285, respectively, and the UART, the USB and the FeliCa interface can be applied to interfaces with the external system 291 (for example, a computer).

The internal clock unit 260 includes an RTC (Real Time Clock), a PLL (Phase Locked Loop), a timing generation unit, and WDT (Watch Dog Timer).

The internal clock which is a reference clock inside the platform integrated circuit 100 can be generated by the PLL based on a oscillation signal of the crystal oscillator 293 input from outside of the platform integrated circuit 100. The timing generation unit generates operation timing for each component of the platform integrated circuit 100. For example, a change timing for the time division multiplexing in the multiplexer 214 is generated for selecting the analog input signals which are from the PGA 211, the amplifier A212 and the amplifier B213.

The power supply unit 270 includes a DC/DC converter, a regulator, and a voltage detector. According to these components, the power supply unit 270 transforms the voltage provided from external power supply 292 into a stable internal electrical power source voltage which is a desired voltage value.

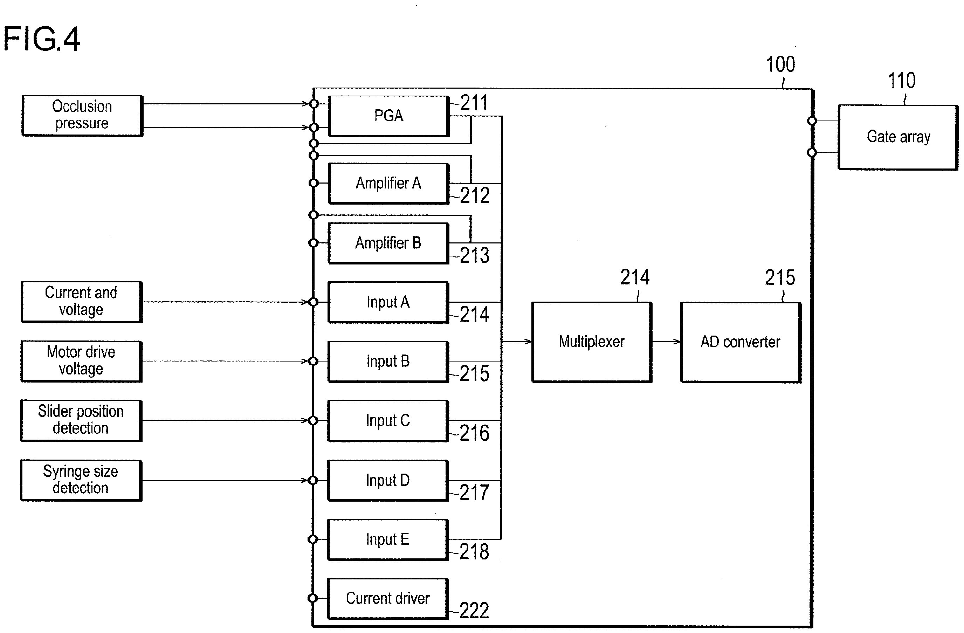

Next, manners of use of the platform integrated circuit 100 in the medical devices of the present embodiment will be explained with reference to FIG. 4 through FIG. 7.

As described above, in cases where the platform integrated circuit 100 is uses for the syringe pump and the infusion pump as the medical device 10, the gate array 110 which complements the arithmetic function of the CPU 221 of the platform integrated circuit 100 is connected to the platform integrated circuit 100. Some functional blocks of the medical devices are omitted in FIG. 4 through FIG. 7.

FIG. 4 is a drawing illustrating a manner of use of the platform integrated circuit 100 in a syringe pump.

The syringe pump is a medical device which has a main purpose to perform nutrition and blood transfusion to a patient, and to inject medical fluid such as a chemotherapeutic drug and an anesthetic drug in high accuracy for relatively long time in an intensive care unit (ICU) etc. and the syringe pump has a superior function of medical fluid flow control in comparison with other types of pumps.

The occlusion pressure detected by the occlusion pressure sensor included in the syringe pump is input into the PGA211 as a differential voltage signal (detection signal) to amplify, and is input into the multiplexer 214 as a signal for one channel. The gain of the PGA 211 is set to a suitable value by the DA converter 223.

Respective signals of power supply voltage (output voltage of a secondary battery), motor drive voltage, slider position detection, and syringe size detection are input into inputs A-D which are input units of the platform integrated circuit 100, respectively, and each of signals is input into the multiplexer 214 as a signal for one channel.

The amplified occlusion pressure and each signal of the power supply voltage, the motor drive voltage, the slider position detection, and the syringe size detection are subjected to the time division multiplexing by the multiplexer 214, and are subjected to the digital conversion by the AD converter 215. Each signal which is subjected to the digital conversion is stored in the external flash memory 284 through the SPI 121, and can be utilized as the signal for control and a failure detection signal of the syringe pump.

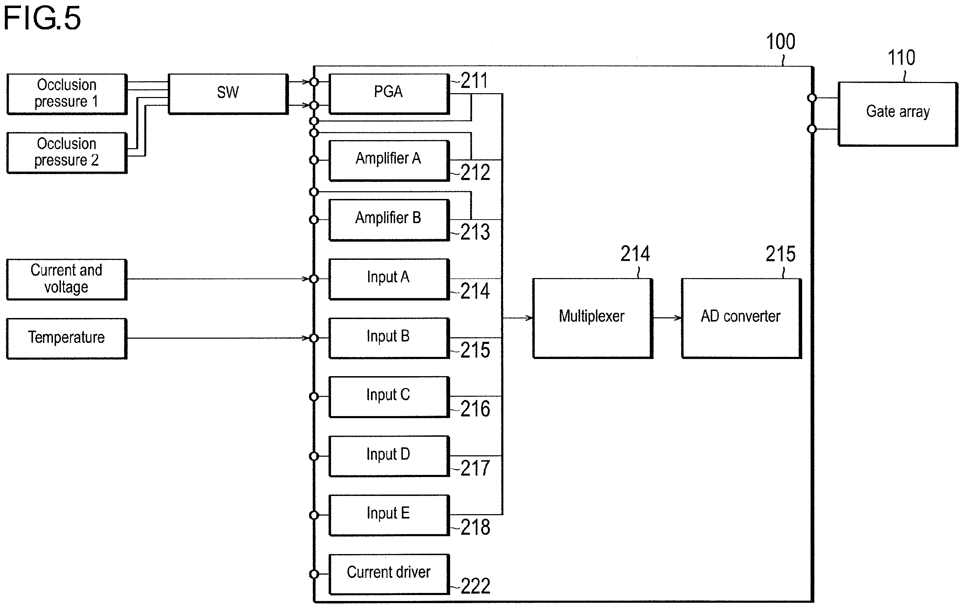

FIG. 5 is a drawing illustrating a manner of use of the platform integrated circuit 100 in an infusion pump.

The infusion pump is a medical device for dosing a drug for the patient continuously with a predetermined dosage for intravenous drip per hour, and for example, it is used to give an intravenous drip injection correctly to the patient after an operation.

With respect to the occlusion pressure 1 and occlusion pressure 2 detected by two occlusion pressure sensors included in the infusion pump respectively, any one of the occlusion pressures selected by a switch SW is input into the PGA 211 as the differential voltage signal (detection signal) to be amplified by the PGA 211, and after the amplification, is input into the multiplexer 214 as a signal for one channel. The gain of the PGA 211 is set to a suitable value by the DA converter 223.

Respective signals of voltage and temperature is input into the input A and the input B which are the input units of the platform integrated circuit 100, and each signal is input into the multiplexer 214 as a signal for one channel.

The two amplified occlusion pressures and each signal of the power supply voltage (output voltage of the secondary battery) and temperature are subjected to the time division multiplexing by the multiplexer 214, and are subjected to the digital conversion by the AD converter 215. Each signal which is subjected to the digital conversion is stored in the external flash memory 284 through the SPI, and can be utilized as a control signal and a failure detection signal of the infusion pump.

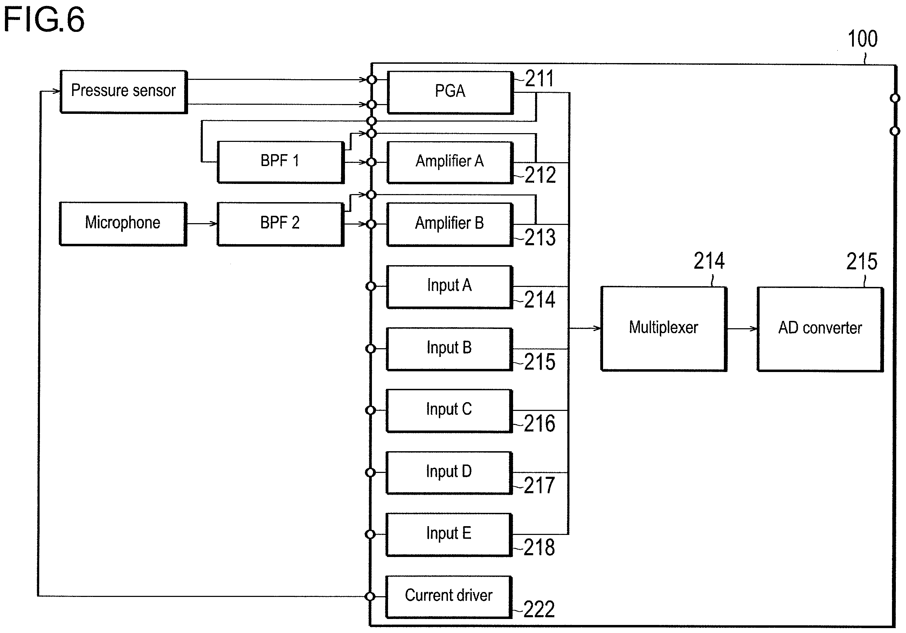

FIG. 6 is a drawing illustrating a manner of use of the platform integrated circuit 100 in a sphygmomanometer.

The sphygmomanometer is a medical device for measuring human blood pressure.

The pressure signal (detection signal) detected by the pressure sensor included in the sphygmomanometer is input into the PGA 211 as an analog differential voltage signal to be subjected to differential amplification by the PGA 211, and after that, is input into the multiplexer 214 as a signal for one channel. The gain of the PGA 211 is set to a suitable value by the DA converter 223.

Furthermore, the pressure signal which is subjected to the differential amplification by the PGA 211 is amplified and filtered by a bandpass filter BPF1 which constitutes a bandpass filter with the amplifier A212, and after that, is input into the multiplexer 214 as a signal for one channel. By filtering the pressure signal by the bandpass filter BPF1, it is possible to extract only pulse wave component which can be directly utilized for blood pressure measurement from the pressure signal.

The pressure signal from the pressure sensor can be utilized for oscillometric blood pressure measurement.

The voice signal (detection signal of voltage) from the microphone is amplified and filtered by a bandpass filter BPF2 which constitutes a bandpass filter with the amplifier B213, and after that, is input into the multiplexer 214 as a signal for one channel.

The voice signal from the microphone can be utilized for the blood pressure measurement by a Korotkov's sound method.

The current driver 222 applies current to the pressure sensor.

Inputs A to E which are input units of the platform integrated circuit 100 may input other signals regarding a measurement of value of the blood pressure.

Each of signals of the amplified pressure, further amplified and filtered pressure and voice is subjected to the time division multiplexing by the multiplexer 214, and are subjected to the digital conversion by the AD converter 215. Each signal which is subjected to the digital conversion is stored in the external flash memory 284 through the SPI, and can be utilized as the measurement result (detection result) of the sphygmomanometer, a control signal and a failure detection signal.

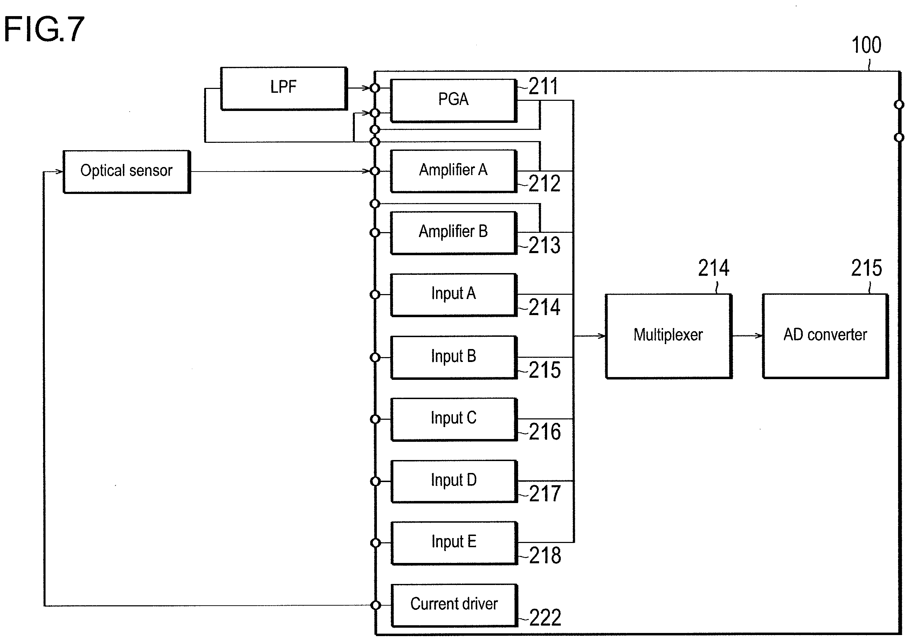

FIG. 7 is a drawing illustrating a manner of use of the platform integrated circuit 100 in a blood glucose meter.

The blood glucose meter is a medical device for measuring human blood glucose level.

A light signal (detection signal of current) detected by a blood glucose sensor included in the blood glucose meter is subjected to a current/voltage conversion and amplified by the amplifier A212 which constitutes transimpedance amplifier with an external resistor, and after that, is input into the multiplexer 214 as one channel.

The blood glucose sensor (optical sensor) includes an LED (Light emitting Diode) and a PD (Photo Diode).

Furthermore, the light signal amplified by the amplifier A212 is input into the PGA 211 as one of differential signals and is input into the PGA 211 through the low pass filter LPF as another of the differential signals.

The PGA 211 amplifies a difference between the light signal input through the LPF and the light signal input without passing through the LPF. Accordingly, the high frequency component utilized for measurement of the blood glucose level can be extracted with high precision from the light signals. The signal amplified by the PGA 211 is input into the multiplexer 214 as one channel. The gain of the PGA 211 is set to a suitable value by the DA converter 223.

The current driver 222 applies current to the LED which constitutes the blood glucose sensor.

Inputs A to E which are input units of the platform integrated circuit 100 may input other signals regarding a measurement of the blood glucose level. For example, a temperature signal may be input from a thermistor which measures temperature for calibration of the blood glucose level.

The amplified light signal and the light signal extracted the high frequency component is subjected to the time division multiplexing by the multiplexer 214, and are subjected to the digital conversion by the AD converter 215. Each signal which is subjected to the digital conversion is stored in the external flash memory 284 through the SPI, and can be utilized as the measurement result (detection result) of the blood glucose meter, a control signal and a failure detection signal. Although the optical sensor is exemplified as the blood glucose sensor used in the blood glucose meter, it is not limited to this sensor but an electrode sensor utilizing amperometry and so on may be applied.

In this way, according to the integrated circuit and the medical devices using the integrated circuit of the present embodiment, a group of common components which are used for a plurality of medical devices respectively is communalized as a platform integrated circuit, while the functional complement integrated circuit is used together with the platform integrated circuit for high performance medical devices. Accordingly, it is possible to communalize the components of a plurality of medical devices, to expand coverage of target medical devices, and to reduce the chip size of the common integrated circuit, and therefore, it is possible to achieve a further cost reduction of the components for medical devices and the medical device using the same.

The integrated circuit and the medical devices using the integrated circuit according to the embodiments of the present invention has been explained, but the present invention is not limited to the above-described embodiments.

For example, it has been explained in the above-described embodiments that the integrated circuit is commonly used to four medical devices of the syringe pump, the infusion pump, the blood glucose meter and the sphygmomanometer, but the integrated circuit may be commonly used to other medical devices, and the number of the medical devices commonly used is not limited.

Moreover, it has been explained in the above-described embodiments that the arithmetic function of the CPU is exemplified as a function which is a part of functions of the integrated circuit and is a subject to be complemented by the functional complement integrated circuit, but the functional complement integrated circuit may complement a function of a buzzer controller or a LCD driver, for example.

It has been explained in the above-described embodiments that the gate array which is general-purpose product is exemplified as the functional complement integrated circuit, but the functional complement integrated circuit may be a semi-custom standard cell and may be a full custom ASIC.

### Explanation of reference numeral

- 10, 11: medical device,
- 100: platform integrated circuit,
- 110: gate array,
- 120: sensor,
- 210: amplification unit,
- 220: control unit,
- 230: arithmetic unit,
- 240: memory unit,
- 250: input-output unit,
- 260: internal clock unit,
- 270: power supply unit,
- 281: buzzer,
- 282: LCD,
- 283: voice amplifier and speaker,
- 284: flash memory,
- 285: battery level indicator,
- 291: external system,
- 292: power supply, and
- 293: crystal oscillator.

## Claims

1. An integrated circuit (100) configured to be used for a plurality of medical devices (10, 11), wherein the plurality of medical devices comprises a first group of medical devices and a second group of medical devices, the medical devices of the second group requiring a higher arithmetic processing performance than the medical devices of the first group,
**characterized in that**
the integrated circuit (100) is connectable to a functional complement integrated circuit (110) which complements a part of functions of the integrated circuit (100),
the integrated circuit (100) is configured to operate by itself the first group of medical devices, and
the integrated circuit (100) is configured to operate the second group of medical devices in cooperation with the functional complement integrated circuit (110) when said functional complement integrated circuit is connected to the integrated circuit (100).

2. The integrated circuit (100) as claimed in claim 1, wherein
the integrated circuit comprises an arithmetic unit, and
the functional complement integrated circuit connected to the integrated circuit (100) complements arithmetic functions of the integrated circuit (100) by a parallel processing with the arithmetic unit (230).

3. The integrated circuit (100) as claimed in claim 1 or 2, wherein the integrated circuit (100) inputs a detection signal from a sensor (120) included in the medical device (10, 11).

4. The integrated circuit (100) as claimed in claim 3, wherein the plurality of medical devices (10, 11) includes a syringe pump, an infusion pump, a blood glucose meter and a sphygmomanometer.

5. The integrated circuit (100) as claimed in claim 3 or 4, further comprising:
an amplification unit (210) which amplifies the detection signal from the sensor (120),
wherein the arithmetic unit (230) receives the detection signal amplified by the amplification unit and performs a calculation of a detection result or an arithmetic process for a control of the medical device (10, 11) according to an internal program.

6. The integrated circuit (100) as claimed in claim 4, wherein the syringe pump and the infusion pump are included in the second group of medical devices, and the blood glucose meter and the sphygmomanometer are included in the first group of medical devices.

## Patentansprüche

1. Integrierte Schaltung (100), die dazu konfiguriert ist, für eine Vielzahl von medizinischen Einrichtungen (10, 11) verwendet zu werden, wobei die Vielzahl von medizinischen Einrichtungen eine erste Gruppe von medizinischen Einrichtungen und eine zweite Gruppe von medizinischen Einrichtungen umfasst, wobei die medizinischen Einrichtungen der zweiten Gruppe eine höhere arithmetische Verarbeitungsleistungsfähigkeit als die medizinischen Einrichtungen der ersten Gruppe erfordern,
**dadurch gekennzeichnet, dass**
die integrierte Schaltung (100) mit einer integrierten Funktionsergänzungsschaltung (110) verbindbar ist, die einen Teil der Funktionen der integrierten Schaltung (100) ergänzt,
die integrierte Schaltung (100) dazu konfiguriert ist, die erste Gruppe von medizinischen Einrichtungen selbst zu betreiben, und
die integrierte Schaltung (100) dazu konfiguriert ist, die zweite Gruppe von medizinischen Einrichtungen in Zusammenarbeit mit der integrierten Funktionsergänzungsschaltung (110) zu betreiben, wenn die integrierte Funktionsergänzungsschaltung mit der integrierten Schaltung (100) verbunden ist.

2. Integrierte Schaltung (100) gemäß Anspruch 1, wobei
die integrierte Schaltung eine Arithmetikeinheit umfasst, und
die integrierte Funktionsergänzungsschaltung, die mit der integrierten Schaltung (100) verbunden ist, arithmetische Funktionen der integrierten Schaltung (100) durch eine parallele Verarbeitung mit der Arithmetikeinheit (230) ergänzt.

3. Integrierte Schaltung (100) gemäß Anspruch 1 oder 2, wobei die integrierte Schaltung (100) ein Erfassungssignal von einem Sensor (120), der in der medizinischen Einrichtung (10, 11) umfasst ist, erhält.

4. Integrierte Schaltung (100) gemäß Anspruch 3, wobei die Vielzahl von medizinischen Einrichtungen (10, 11) eine Spritzenpumpe, eine Infusionspumpe, ein Blutzuckermessgerät und ein Sphygmomanometer umfasst.

5. Integrierte Schaltung (100) gemäß Anspruch 3 oder 4, weiterhin mit:
einer Verstärkungseinheit (210), die das Erfassungssignal von dem Sensor (120) verstärkt,
wobei die Arithmetikeinheit (230) das Erfassungssignal, das durch die Verstärkungseinheit verstärkt wird, empfängt, und eine Berechnung eines Erfassungsergebnisses oder einen arithmetischen Prozess für eine Steuerung der medizinischen Einrichtung (10, 11) gemäß einem internen Programm durchführt.

6. Integrierte Schaltung (100) gemäß Anspruch 4, wobei die Spritzenpumpe und die Infusionspumpe in der zweiten Gruppe von medizinischen Einrichtungen umfasst sind, und das Blutzuckermessgerät und das Sphygmomanometer in der ersten Gruppe von medizinischen Einrichtungen umfasst sind.

## Revendications

1. Circuit intégré (100) configuré pour être utilisé pour une pluralité de dispositifs médicaux (10, 11), la pluralité de dispositifs médicaux comprenant un premier groupe de dispositifs médicaux et un second groupe de dispositifs médicaux, les dispositifs médicaux du second groupe nécessitant une performance de traitement arithmétique plus élevée que les dispositifs médicaux du premier groupe,
**caractérisé en ce que** le circuit intégré (100) peut être connecté à un circuit intégré de complément fonctionnel (110) qui complète une partie des fonctions du circuit intégré (100),
le circuit intégré (100) étant configuré pour faire fonctionner à lui seul le premier groupe de dispositifs médicaux, et
le circuit intégré (100) étant configuré pour faire fonctionner le second groupe de dispositifs médicaux en coopération avec le circuit intégré à complément fonctionnel (110) lorsque ledit circuit intégré à complément fonctionnel est connecté au circuit intégré (100).

2. Circuit intégré (100) selon la revendication 1,
le circuit intégré comprenant une unité arithmétique, et
le circuit intégré à complément fonctionnel connecté au circuit intégré (100) complétant les fonctions arithmétiques du circuit intégré (100) par un traitement parallèle avec l'unité arithmétique (230).

3. Circuit intégré (100) selon la revendication 1 ou 2, le circuit intégré (100) entrant un signal de détection provenant d'un capteur (120) inclus dans le dispositif médical (10, 11).

4. Circuit intégré (100) selon la revendication 3, la pluralité de dispositifs médicaux (10, 11) comprenant un pousse-seringue, une pompe à perfusion, un lecteur de glycémie et un sphygmomanomètre.

5. Circuit intégré (100) selon la revendication 3 ou 4, comprenant en outre :
une unité d'amplification (210) qui amplifie le signal de détection provenant du capteur (120),
l'unité arithmétique (230) recevant le signal de détection amplifié par l'unité d'amplification et réalisant un calcul d'un résultat de détection ou un processus arithmétique pour une commande du dispositif médical (10, 11) selon un programme interne.

6. Circuit intégré (100) selon la revendication 4, le pousse-seringue et la pompe à perfusion étant inclus dans le second groupe de dispositifs médicaux, et le lecteur de glycémie et le sphygmomanomètre étant inclus dans le premier groupe de dispositifs médicaux.
